(19) Europäisches Patentamt / European Patent Office / Office européen des brevets

(11) **EP 2 088 953 B1**

(12) **EUROPÄISCHE PATENTSCHRIFT**

(45) Veröffentlichungstag und Bekanntmachung des Hinweises auf die Patenterteilung:
**06.04.2011 Patentblatt 2011/14**

(21) Anmeldenummer: **08706731.0**

(22) Anmeldetag: **09.01.2008**

(51) Int Cl.:
**A61B 18/20** *(2006.01)* **C12M 1/34** *(2006.01)*

(86) Internationale Anmeldenummer:
**PCT/DE2008/000026**

(87) Internationale Veröffentlichungsnummer:
**WO 2008/086772 (24.07.2008 Gazette 2008/30)**

(54) **LASERDOSIMETRIE FÜR DIE OPTOPERFORATION EINZELNER ZELLEN**

LASER DOSIMETRY FOR THE LASER PERFORATION OF INDIVIDUAL CELLS

DOSIMÉTRIE LASER POUR L'OPTOPERFORATION DE CELLULES INDIVIDUELLES

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **18.01.2007 DE 102007003600**

(43) Veröffentlichungstag der Anmeldung:
**19.08.2009 Patentblatt 2009/34**

(73) Patentinhaber: **Universität zu Lübeck**
**23538 Lübeck (DE)**

(72) Erfinder: **VOGEL, Alfred**
**23568 Lübeck (DE)**

(74) Vertreter: **Biehl, Christian et al**
**Boehmert & Boehmert**
**Anwaltssozietät**
**Niemannsweg 133**
**24105 Kiel (DE)**

(56) Entgegenhaltungen:
- **VOGEL A ET AL: "Mechanisms of femtosecond laser nanosurgery of cells and tissues" APPLIED PHYSICS B ; LASERS AND OPTICS, SPRINGER-VERLAG, BE, Bd. 81, Nr. 8, 1. Dezember 2005 (2005-12-01), Seiten 1015-1047, XP019337596 ISSN: 1432-0649**
- **TUZIUTI T ET AL: "Spatial study on a multibubble system for sonochemistry by laser-light scattering." ULTRASONICS SONOCHEMISTRY JAN 2005, Bd. 12, Nr. 1-2, Januar 2005 (2005-01), Seiten 73-77, XP002480408 ISSN: 1350-4177**
- **RAU KAUSTUBH R ET AL: "Pulsed laser microbeam-induced cell lysis: Time-resolved imaging and analysis of hydrodynamic effects" BIOPHYSICAL JOURNAL, Bd. 91, Nr. 1, Juli 2006 (2006-07), Seiten 317-329, XP002480409 ISSN: 0006-3495**
- **RAU KAUSTUBH R ET AL: "Investigation of laser-induced cell lysis using time-resolved imaging" APPLIED PHYSICS LETTERS, AIP, AMERICAN INSTITUTE OF PHYSICS, MELVILLE, NY, Bd. 84, Nr. 15, 12. April 2004 (2004-04-12), Seiten 2940-2942, XP012061117 ISSN: 0003-6951**
- **CHEN XIAO ET AL: "Shock-wave propagation and cavitation bubble oscillation by Nd:YAG laser ablation of a metal in water." APPLIED OPTICS 1 JUN 2004, Bd. 43, Nr. 16, 1. Juni 2004 (2004-06-01), Seiten 3251-3257, XP002480410 ISSN: 0003-6935**

EP 2 088 953 B1

## Beschreibung

Das Einbringen eines nicht-membrangängigen Fremdmaterials (z.B. Nukleinsäuremoleküle, Chromosomen, Organellen, Nanopartikel, Proteine, Farbstoffe, pharmazeutische Wirkstoffe) in Zellen stellt ein weit verbreitetes Problem in der Zellbiologie dar. Besonders schwierig gestaltet sich das gezielte Einbringen der o. g. Substanzen in ausgewählte Einzelzellen innerhalb einer Zellpopulation.

Während leicht zu handhabende und daher in den Laboren auch weit verbreitete Methoden zur Überwindung der Zellmembran, wie z.B. die Elektroporation (d.h. die transiente Permeabilisierung der Zellmembran durch Spannungspulse) oder der Einsatz von Liposomen (Lipidvesikel, in deren Inneren sich das einzubringende Fremdmaterial befindet und die mit der Zellmembran verschmelzen) in der Regel unspezifisch auf eine Vielzahl von Zellen gleichzeitig wirken, stand bis vor kurzem für die Manipulation von Einzelzellen im Wesentlichen nur das sowohl apparativ sehr aufwendige als auch von der Handhabung her höchst anspruchsvolle Verfahren der Mikroinjektion zur Verfügung. Hierbei wird das Fremdmaterial mit Hilfe einer Mikrokapillare direkt in den Zellkern bzw. ins Cytoplasma der Zelle injiziert. Das Verfahren besitzt eine Effizienz von nahezu 100 %. Allerdings können nur relativ wenige Zellen in einem praktikablen Zeitraum manipuliert werden.

Der Fortschritt in der Laser-Nanochirurgie führte in den letzten Jahren zu der Entwicklung der Laser-vermittelten Permeabilisierung der Zellmembran (Optoperforation, auch als Laser- oder Photoperforation bezeichnet), um auf diesem Wege das Einbringen von Fremdmaterial in ausgewählte Einzelzellen zu ermöglichen. Mittels einer entsprechenden Mikroskopanordnung, wie z.B. in Stevenson et al. (2006, Optics Express, Vol. 14, No. 16, pp. 7125-33) dargestellt, kann die Zellmembran von Einzelzellen mit gepulstem Laserlicht bestrahlt werden. Am Ort des Auftreffens des Laserlichts auf die Zellmembran kommt es bei ausreichender Strahlungsintensität zur Bildung von Kavitationsblasen. Hierbei werden die Blasen in einem nominell transparenten Medium durch Multiphotonenabsorption generiert, wobei die Zwei-Photonen-Absorption eine wichtige Rolle spielt (s. Stevenson et al.). Es wird inzwischen davon ausgegangen, dass bei Verwendung von einzelnen Laserpulsen oder von Pulsserien mit Repetitionsraten ≤ 1 MHz das einzubringende Fremdmaterial nur dann effektiv von der Zielzelle aufgenommen werden kann, wenn es während der Bestrahlung zur Bildung dieser Kavitationsblasen kommt (Vogel et al., 2005, Applied Physics B 81, pp. 1015-47). Andererseits wirken sich zu hohe Strahlungsdosen und somit zu große Kavitationsblasen negativ auf die Viabilität der Zielzelle aus, es kommt dann in der Folge zu einer erhöhten Sterblichkeit der behandelten Zellen, wodurch wiederum die Effektivität der Methode beeinträchtigt wird.

Die Applikation der optimalen Strahlungsdosis, bei der eine effektive Permeabilisierung der Zellmembran bei einer möglichst hohen Überlebensrate der bestrahlten Zellen gewährleistet ist, stellt somit die entscheidende Voraussetzung für den Erfolg der Methode dar. Die optimale Strahlungsdosis ist dabei in hohem Maße abhängig von der jeweils zu bestrahlenden Probe. Je nach Zell- oder Gewebetyp, physiologischen Zustand der Zellen und dem die Zellen umgebenden Medium bzw. Umfeld müssen die Laserparameter jeweils individuell angepasst werden, d.h. es ist eine Kalibrierung für die Laserbearbeitung und die Überwachung dieser Bearbeitung erforderlich.

Als Indikator für die jeweiligen durch das applizierte Laserlicht erzielten Effekte könnte hier die Größe der entstehenden Kavitationsblasen dienen, wobei, wie von Vogel et al. (2005, Applied Physics B, 81, pp. 1015-47) vorgeschlagen, die Bestimmung der Blasengröße über die Messung der jeweiligen Blasenoszillationzeit (d. h. der Blasenlebensdauer) erfolgen kann. Als ein möglicher Zugang zum Online-Monitoring der Blasengröße oder -lebensdauer wird in dieser Arbeit die Streuung von Licht an im Laserfokus gebildeten Blasen erwähnt; wie dieses Ziel konkret erreicht werden soll wird jedoch nicht beschrieben.

In der DE 103 31 792 A1 wird ein Laser mit Dosimetriesteuerung offenbart, bei dem das erste Auftreten von Blasen innerhalb eines Gewebes u. a. interferometrisch über die Änderung des Brechungsindex erfasst werden kann. Dies dient dazu, die Leistung des Lasers derart zu modulieren, dass größtenteils dicht oberhalb der Blasenbildungsschwelle bestrahlt werden kann. Hierbei wird jedoch lediglich das Auftreten der Blasen detektiert, eine Bestimmung der Lebensdauer der Blasen und Rückschlüsse hieraus auf die Blasengröße werden nicht beschrieben.

In der Dissertation von Jörg Neumann "Mikroskopische Untersuchungen zur laser-induzierten Blasenbildung und -dynamik an absorbierenden Mikropartikeln" (Universität zu Lübeck, 2005) werden lineare Absorptionsprozesse an Mikropartikeln (Absorber, z.B. Pigmente) untersucht, wie sie bei der Lasertherapie absorbierender Zellschichten, insbesondere am Augenhintergrund, auftreten. Eine wichtige Zielsetzung der Arbeit liegt im Aufzeigen des Einflusses dieser Partikel etwa auf die Blasendynamik für verschiedene Energien der die Blasen induzierenden Strahlung. Die Arbeit offenbart eine Messung der Lebensdauer laser-induzierter Mikroblasen mittels der Streuung eines Probelaserstrahls im Laserfokus der blaseninduzierenden Strahlung. Dabei werden Blasen mit Lebensdauern um 100 ns detektiert. Das Verfahren ist aber nicht für die Anwendung in Echtzeit ausgebildet, und es erfolgt keine Umrechnung in Blasengrößen und damit auch keine vollständige Einschätzung des durch die Blasen erzielten Schadens an Zellen. Zwar thematisiert die Dissertation auch eine Online-Dosimetriekontrolle mit interferometrischen Methoden in Rückstreuung, aber diese bleibt in ihrer Anwendbarkeit auf Blasen der Größenordnung Mikrometer beschränkt und zielt vorrangig auf die Detektion allein des Auftretens solcher Blasen ab.

Ob sich eine quantitative Messung der Blasenlebensdauer mittels eines Durchlichtverfahrens (z.B. via Streuung) überhaupt in einem nominell transparenten Medium ohne Absorberpartikel, in dem die Blasen einzeln durch Mehrphotonenabsorption entstehen und Durchmesser deutlich unter 100 nm aufweisen können, insbesondere als Methode zum Online-Monitoring realisieren lässt, war bis jetzt nicht bekannt.

Es ist nun die Aufgabe der Erfindung, ein Verfahren zur Optoperforation einzelner Zellen mittels gepulster Laserstrahlung anzugeben, bei dem der Grad der Zellmembran-Permeabilisierung derart kontrolliert werden kann, dass die Effizienz der Fremdkörperaufnahme in die bestrahlte Zelle möglichst maximal ist und zugleich die Viabilität der Zellen nicht unnötig beeinträchtigt wird.

Die Aufgabe wird erfindungsgemäß durch ein Verfahren zur Optoperforation der Zellmembran einer Zelle durch Applikation von Laserlichtpulsen mit den in Anspruch 1 dargestellten Schritten gelöst. Die Unteransprüche geben vorteilhafte Weiterbildungen des Verfahrens nach Anspruch 1 an.

Wie bereits erwähnt, kann die Aufnahme von Fremdkörpern in die Zelle durch die Beobachtung der Blasenoszillation während der Pulslaserapplikation realisiert werden. Blasenbildung findet nicht immer unter der Laserapplikation statt, sondern es ist ebenso möglich, dass das durch nichtlineare Absorption von Laserpulsen erzeugte Plasma (insbes. Freie Elektronen) im Applikationsbereich nicht ausreicht, um eine Blase aufschwingen zu lassen. Ein derartiges Plasma zerstört jedoch u. a. chemische Bindungen in der Zellmembran und kann durch Akkumulation der Effekte vieler Laserpulse die Zelle sozusagen auf chemischem Wege öffnen. Bei Blasenbildung erfolgt die lokale Öffnung der Zellmembran hingegen eher auf thermomechanischem Wege wofür eine weitaus geringere Anzahl von Laserpulsen ausreicht. Darüber hinaus ist es durch Perforation der Zellmembran mit dem erfindungsgemäßen Verfahren ebenso gut möglich, Stoffe aus der Zelle auszuschleusen oder über die Zellmembran aufgebaute (chemische) Potenziale kollabieren zu lassen. Die folgenden Ausführungen beziehen sich deshalb lediglich beispielhaft auf die erfindungsgemäße Anwendung zur Aufnahme von Stoffen in die Zelle.

Grundlage der Erfindung ist die Erkenntnis, dass die Zellmembraneröffnung mittels einzelner Laserpulse oder Pulsserien mit Repetitionsraten < 1 MHz immer mit transienter Blasenbildung im Laserfokus einhergeht. Die Blasen haben dann Lebensdauern im Bereich von Nanosekunden bis Mikrosekunden - im Unterschied zu den langlebigen Blasen, die bei Photoperforation mit Femtosekunden-Oszillatorpulsen (> 1 MHz Repetitionsrate) entstehen, wenn man die Laserleistung überdosiert.

Im Folgenden soll stets darauf abgezielt werden, dass Blasenbildung möglich ist. Typische Laserparameter, die dies erlauben, sind insbesondere Pulsdauern im Piko- bis Femtosekundenbereich (ps oder fs), Repetitionsraten unter 1 MHz, bevorzugt um 1 kHz, und Pulsenergien in den Größenordnungen 1 bis 10.000 nJ. Die Blasenbildung bei Fokussierung des Pulslasers auf die Zellmembran tritt in Erscheinung, sobald eine dafür ausreichenden Pulsenergie eingestellt wird. Dies lässt sich allerdings nicht durch eine feste, vorgewählte Energieeinstellung erreichen, weil sich Fokusqualität (Fleckgröße), Energieverluste auf dem Weg zum Laserfokus und die Absorptionseigenschaften der Zielstruktur von Fall zu Fall ändern. Das reproduzierbare Erzeugen von Blasen, die idealerweise stets dieselbe Größe aufweisen sollen - und damit dasselbe Beschädigungspotenzial für die Zellmembran - erfordert deshalb eine adaptive Pulslasersteuerung, die sich auf die Beobachtung der Blasengröße stützt. Welche Größe optimal ist, hängt von den jeweiligen Zelleigenschaften ab.

Erfindungsgemäß wird daher die Blasenbildung kontinuierlich überwacht und im Zeitregime aufgezeichnet. Dabei wird die Zeitspanne zwischen dem ersten Aufschwingen der Blase bis zu ihrem ersten Kollaps (i. F. bezeichnet als Oszillationszeit), möglichst genau gemessen. Dies wird möglich durch das Detektieren der Streuung eines Probelichtstrahls, vorzugsweise eines Probelaserstrahls. Der Probelichtstrahl soll selbst keine Wirkung auf Zellen oder umgebendes Medium entfalten, sondern dient allein zur Überwachung der optischen Eigenschaften des Materials im Pulslaserfokus. Vorzugsweise wird ein leistungsschwacher cw-Laser verwendet mit einer Hauptemissionswellenlänge, die sich wesentlich von der des Pulslasers unterscheidet Es hat sich erwiesen, dass Wellenlängen aus dem Nahinfrarot-Spektrum, z.B. 780 nm, besonders geeignet sind, weil sie die mikroskopische Beobachtung der Zellen nicht stören.

Der Probelaserstrahl muss dazu den Bereich des Pulslaserfokus durchqueren, in dem das Pulslaserlicht - bevorzugt unmittelbar an der Zellmembran - für die Blasenbildung sorgt. Es ist dafür praktisch, den Probelaserstrahl in den Strahlengang des Pulslaserlichts einzuspiegeln. Dies ist mit einem dichroitischen Spiegel möglich, ebenso wie das Auskoppeln des Probelaserstrahls nach dem Durchqueren des Fokus. Der ausgekoppelte Probelaserstrahl wird auf einen Detektor geführt, der die Lichtintensität fortlaufend registriert.

Schwankungen in der Lichtintensität des Probelasers sind auf Streuprozesse im Pulslaserfokus zurückzuführen, wenn eine Blase oszilliert. Bei sehr kleinen Blasen sind diese Schwankungen äußerst schwach und sehr kurzzeitig. Um sie überhaupt messbar zu machen, wird das Streulichtsignal des Probelasers mit einem empfindlichen AC-gekoppelten High-Speed-Photoreceiver (Diode mit Verstärker für den Fotostrom) vorzugsweise mit einer Signalbandbreite von 25 kHz bis 200 MHz detektiert. Durch die AC-Kopplung werden alle „langsamen" (hier bis 25 kHz) Schwankungen aus dem Signal gefiltert. Solche langsamen Schwankungen können leicht aufgrund von Leistungsschwankungen des Probelasers oder durch sonstige externe Einflüsse entstehen. Zudem ist die zu detektierende Blase häufig deutlich kleiner als das Fokusvolumen, wodurch der Streulichtanteil erheblich geringer ist als die Gesamtintensität des durch den Fokus transmittierten Lichtes. Durch die AC-Kopplung werden Gleichanteile aus der Messung entfernt.

Trotzdem bietet der Phtitoreceiver die Möglichkeit, sich auch das DC-Signal anzusehen. Dies ist wichtig für die Grundjustage des Systems, denn der Probelaser muss optimal in den Strahlengang des Pulslasers einjustiert werden, was einem maximalen DC-Signal entspricht. Die hohe Bandbreite und die Empfindlichkeit des AC-Photoreceivers ermöglichen die Detektion kleinster Änderungen im Streulichtsignal des Probelasers. Die Dauer des Streulichtsignals entspricht der Blasenoszillationszeit. Da der Photoreceiver eine Anstiegs- und Abfallzeit von 1.8 ns besitzt, eignet er sich zur Bestimmung von Blasenoszillationszeiten bis unter 5 ns. Die kürzeste bisher gemessene Blasenoszillationszeit betrug 15 ns (an der Schwelle zur Blasenbildung mit Femtosekundenpulsen und Fokussierung mit NA = 0.9), was einem Blasenradius von nur 150 nm entspricht. Da noch Reserven im Messbereich vorhanden sind, ist das System für alle auftretenden Eventualitäten (d.h. noch kleinere Blasen bei maximaler numerischer Apertur des Mikroskopobjektivs von NA = 1.3) gerüstet.

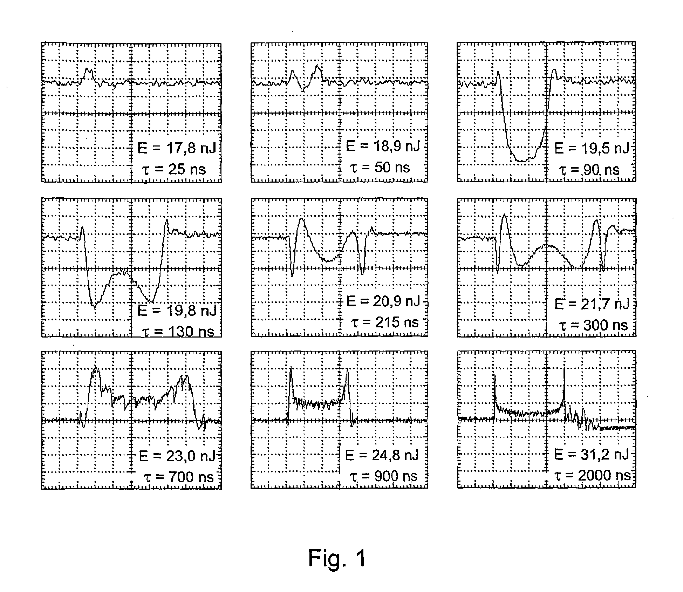

Fig. 1 zeigt eine Auswahl von Messungen der Blasenoszillation mit dem erfmdungsgemäßen Messverfahren wie zuvor beschrieben. Alle Grafiken stellen die gemessene Lichtintensität gegenüber der Zeitachse dar und sind mit Angaben zu verwendeten Pulsenergien E und daraus resultierenden Oszillationszeiten τ versehen. Die Blasenoszillationen sind gekennzeichnet durch signifikante Abweichungen vom ansonsten konstanten Signalverlauf.

Die erfindungsgemäß ermittelte Oszillationszeit der lasererzeugten Blasen kann über die Rayleigh-Beziehung

$$R_{max} = \frac{T}{1.83} \sqrt{\frac{p_0 - p_v}{\rho_0}}$$

in die Maximalausdehnung der Blasen umgerechnet werden. Dabei sind $p_0$ der hydrostatische Druck in der Umgebung der Blase, $p_v$ der Blaseninnendruck und $p_0$ die Dichte des Mediums.

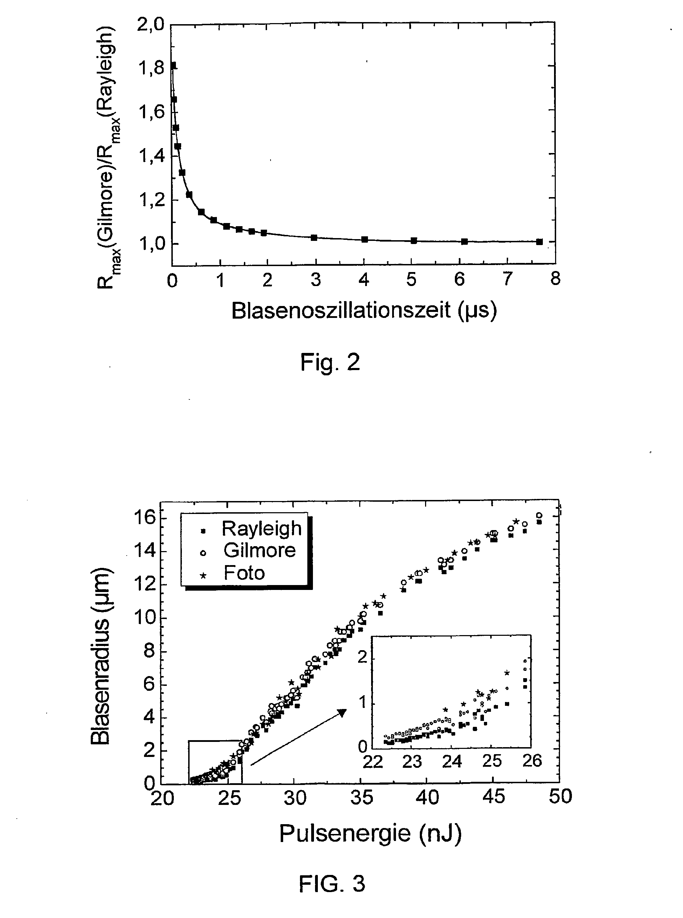

Die Rayleigh-Beziehung ist aus anderen technischen Feldern wohlbekannt, wird aber hier wohl erstmals für nanoskalige Blasen, die bei Laserapplikation entstehen, verwendet. Es zeigt sich denn auch, dass sie zu falschen Ergebnissen führt, da die Oberflächenspannung nicht berücksichtigt wird. Die Oberflächenspannung σ wirkt wie ein Zusatzdruck p = 2σ/R auf die Blase, dessen Amplitude umgekehrt proportional zum Blasenradius R ist. Dieser Zusatzdruck verändert den Zusammenhang zwischen Oszillationszeit und Maximalausdehnung der Blase. Mit Hilfe des Gilmore-Modells, das die Oberflächenspannung mit ihrer Temperaturabhängigkeit berücksichtigt, lassen sich aber Korrekturfaktoren zur Rayleigh-Beziehung errechnen (siehe dazu Fig. 2). Aus Fig. 3 ist zu entnehmen, dass die Rayleigh-Beziehung gerade für sehr kleine Blasen die Durchmesser stark unterschätzt, während das Gilmore-Modell sehr viel bessere Übereinstimmung mit fotografischen Messungen zeigt.

Damit sind nun in guter Näherung die erzeugten Blasengrößen aus den Blasenoszillationszeiten ermittelbar. Dabei ist bemerkenswert, dass bereits Blasenradien bis hinab zu 150 nm optisch nachgewiesen wurden, obwohl der Probelaser eine deutlich größere Wellenlänge aufweist. Die Nachweisgrenze des gegenwärtig verwendeten Fotodetektors mit 200 MHz Bandbreite liegt bei R ≈ 50 nm.

Es wurde bereits erwähnt, dass andere Arbeiten die Blasengröße als einen kritischen Faktor für die Effizienz der zellulären Fremdkörperaufnahme identifiziert haben. Sind die Blasen zu klein, perforieren sie die Zellmembran nicht in ausreichendem Maße, um den einzubringenden Fremdkörpern (typisch sollen Nukleinsäuren, Chromosomen, Proteine, Organelle, Farbstoffe, pharmazeutische Wirkstoffe oder funktionalisierte Nanopartikel in die Zellen eindringen) Zugang zum Zellinnern zu gewähren. Sind die Blasen indes zu groß, werden die Zellmembranen so stark beschädigt, dass sich die Zelle nicht mehr von der Beschädigung erholt und die Bearbeitung nicht überlebt. In der Literatur sind Angaben zwischen 5 und 7,5 Mikrometern als Obergrenze des zur Perforation dienlichen Blasendurchmessers zu finden. Größere Blasen ziehen dort den Zelltod nach sich.

Das für die optimale Transfektionseffizienz geeignete Intervall, in dem die erzeugten Blasendurchmesser liegen sollten, um eine möglichst hohe Zellviabilität zu sichern, kann nicht universell angegeben werden. Es hängt vom gewählten Zelltyp, dem umgebenden Medium und auch von der Art der einzubringenden Fremdkörper ab, da auch diese auf die Laserpulse reagieren können. Dieses optimale Intervall muss jeweils im Einzelfall der in Betracht stehenden Aufgabe bestimmt werden. Als Faustregel kann man davon ausgehen, dass die Blasengröße wenigstens der Größe der Fremdkörper entsprechen sollte, höchstens aber einem Bruchteil des Zelldurchmessers.

Die erforderlichen Kalibrierversuche, mit denen insbesondere die Erfolgsquote des Einbringens der Fremdkörper in die Zellen und die Zellviabilität ggf. auch getrennt untersucht werden, sind Stand der Technik und trotz eines gewissen Aufwandes heute üblich.

Die Erfindung stellt neben der Laserpulsenergie eine zusätzliche Messgröße, die Blasengröße bzw. die Blasenoszillationszeit, zur Verfügung, für die im Zuge der Kalibrierung eines günstiges Intervall aufzufinden ist.

Der besondere Vorteil der Erfindung liegt in der kontinuierlichen Messbarkeit dieser Größe auch während des eigentlichen Bearbeitungsvorganges. In der Tat ist es dadurch nicht nur möglich, die tatsächlich im Pulslaserfokus entstehende Blase praktisch schritthaltend mit der Pulsapplikation zu vermessen, sondern durch geeignete Ansteuerung der Pulslaserquelle kann sogar das Pulslaserlicht modifiziert werden, um ein aktiv kontrolliertes, die Zelle schonendes Bestrahlen zu realisieren.

Aus Fig. 3 ist ebenfalls ersichtlich, dass ein monotoner Zusammenhang zwischen der Pulsenergie der applizierten Strahlung und der beobachtbaren Blasengröße besteht. Von daher ist es erfindungsgemäß zu bevorzugen, die Pulsenergie des Bearbeitungslasers zu steuern. Dies kann zwar prinzipiell über die Kontrolle der Pumpleistung oder über die Rotation einer zwischen Polarisatoren angebrachten λ/2-Platte geschehen, deutlich vorteilhafter ist jedoch die Verwendung eines akustooptischen Modulators, weil dieser ein sehr schnelles Schalten gestattet.

Eine automatisierte Steuerung der Optoperforation könnte folgendermaßen aussehen:

Der Laser zielt auf die Zellmembran oder auf einen vorgewählten Ort in der Nähe der Zellmembran. Es wird nun eine Laserpulsfolge appliziert, bei der die Energie des ersten Pulses unterhalb der zuvor bestimmten oder aus vorangegangenen Eingriffen bekannten Schwelle für die Blasenbildung liegt. Die Pulsenergie wird bei den folgenden Pulsen nach und nach erhöht (ansteigende Pulsenergierampe), bis eine vorgewählte Blasengröße erreicht ist. Die Pulsserie wird nun entweder sofort beendet oder mit konstanter Pulsenergie weitergeführt, bis eine vorgewählte Zahl zusätzlicher Pulse appliziert ist, welche die jeweils gewünschte Blasengröße erzeugen.

Die vorstehende "Pulslaserapplikations-Strategie" ist vorzugsweise durch eine geeignete Rechnerimplementation nach dem Stand der Technik zu realisieren. Sie umfasst insbesondere das wiederholte Auslesen des AC-Photodetektors, die schritthaltende Interpretation der Messdaten hinsichtlich der aktuell erzeugten Blasengröße und eine durch Programmparameter konditionierte Ansteuerung der die Pulsenergie variierenden Einheit, bevorzugt eines akustooptischen Modulators.

Die obige Strategie und der Ansatz zu ihrer Umsetzung sind im Kern der Druckschrift DE 103 31 792 A1 entliehen. Allerdings gibt diese keinerlei Hinweis auf eine Möglichkeit, wie die Größe einzelner erzeugter Blasen zu bestimmen wäre. Bei der DE 103 31 792 A1 geht es um die Laserbehandlung von Gewebeschichten, mithin um die simultane Bestrahlung einer großen Zahl sehr unterschiedlicher Zellen, so dass sich eine solche Frage dort nicht unmittelbar stellt.

Die Optoperforation bestimmter einzelner Zellen stellt insofern höhere Anforderungen an die Dosimetriekontrolle. Die hierfür zweckdienliche Blasengröße muss und kann sehr viel genauer bestimmt und reproduzierbar erzeugt werden, als der Stand der Technik dies bislang zuließ.

Es sollte abschließend noch erwähnt werden, dass die Verwendung der Rayleigh-Beziehung und/oder der Korrekturen des Gilbert-Modells nicht zwingend erforderlich sind, um eine funktionsfähige Dosimetriekontrolle zu realisieren. Hierfür reicht allein schon die Messung der Blasenoszillationszeit völlig aus sofern das Verfahren auf die Oszillationszeit kalibriert wird. Die Übersetzungen in Blasengrößen waren und sind jedoch für das Verständnis der erzielten Effekte sehr nützlich und können natürlich auch bei der adaptiven Kontrolle des Pulslasers ohne großen Aufwand errechnet und protokolliert werden. Es kann von Vorteil sein, diese ermittelten Blasengrößen im Zusammenhang z.B. mit der Kenntnis des bearbeiteten Zelltyps zur algorithmischen Optimierung der Lasersteuerung zu verwenden, etwa zur Beschleunigung des Anstiegs der Pulsenergierampe.

**Patentansprüche**

1.    Verfahren zur Optoperforation der Zellmembran einer Zelle durch Applikation von Laserlichtpulsen, **gekennzeichnet durch**

    - Fokussieren des Pulslaserlichts auf die zu perforierende Zellmembran,
    - Applizieren einer Folge von Laserpulsen mit vorbestimmter Pulsenergie,
    - Messen der Oszillationszeit von im Laserfokus entstehenden Blasen anhand der im Laserfokus **durch** die Blasen hervorgerufenen Lichtintensitätsänderung eines **durch** den Laserfokus transmittierten Probelaserstrahls und
    - Steigern der Pulsenergie bis zu einem Wert, bei dem die Oszillationszeit der Blasen einen vorbestimmten Wert erreicht.

2.    Verfahren nach Anspruch 1, **dadurch gekennzeichnet, dass** nur Lichtintensitätsänderungen mit einer Dauer von weniger als 40 Mikrosekunden erfasst werden.

3.    Verfahren nach einem der vorhergehenden Ansprüche, **dadurch gekennzeichnet, dass** zur Erzeugung des Probelaserstrahls ein cw-laser mit einer Hauptemissionswellenlänge aus dem NIR-Spektrum verwendet wird.

4.    Verfahren nach einem der vorhergehenden Ansprüche, **dadurch gekennzeichnet, dass** dem Verfahren Vorversuche vorangehen, in denen der Oszillationszeit-Sollwert für einen bestimmten Zelltyp und / oder einen bestimmten von der Zelle aufzunehmenden Stoff in Abhängigkeit von der Zellviabilität nach Applikation einer Pulsfolge mit vorbestimmter Pulsenergie ermittelt wird.

**5.** Verfahren nach einem der vorhergehenden Ansprüche, **dadurch gekennzeichnet, dass** Laserpulse im ps- oder fs-Bereich mit einer Repetitionsrate kleiner als 1 MHz appliziert werden.

**6.** Verfahren nach Anspruch 5, **dadurch gekennzeichnet, dass** die Repetitionsrate etwa 1 kHz entspricht.

**7.** Verfahren nach einem der vorhergehenden Ansprüche, **dadurch gekennzeichnet, dass** die Pulsenergie während des Applizierens einer Folge von Pulsen durch Ansteuerung eines akustooptischen Modulators, den die Pulsfolge durchläuft, von Puls zu Puls verändert wird.

**8.** Verfahren nach einem der vorhergehenden Ansprüche, **dadurch gekennzeichnet, dass** eine Pulsenergie zwischen 1 und 10.000 nJ appliziert wird.

**9.** Verfahren nach einem der vorhergehenden Ansprüche, **dadurch gekennzeichnet, dass** nach Erreichen des Oszillationszeit-Sollwerts eine vorbestimmte Anzahl von Pulsen appliziert wird.

**10.** Verfahren nach einem der vorhergehenden Ansprüche, **dadurch gekennzeichnet, dass** die Zelle in einem Medium angeordnet ist, das einen Stoff zur Aufnahme in die Zelle aufweist, der aus der Gruppe von Nukleinsäuren, Chromosomen, Proteinen, Organellen, Farbstoffen, pharmazeutischen Wirkstoffen und fimktionalisierten Nanopartikeln ausgewählt ist.

**Claims**

**1.** Method of optoperforation of a cell membrane by application of laser pulses,
**characterized by**

- focusing the pulsed laser onto the cell membrane to be perforated,
- applying a series of laser pulses of predetermined pulse energy,
- measuring the oscillation time of the bubbles formed in the laser focus from the change in laser intensity of a test laser beam transmitted through the laser focus and caused by the bubbles in the laser focus and
- increasing the pulse energy to a level at which the oscillation time of the bubbles attains a predetermined value.

**2.** Method according to claim 1, **characterized in that** only laser intensity changes, lasting less than 40 microseconds, are recorded.

**3.** Method according to one of the preceding claims, **characterized in that** a cw-laser with a main emission wavelength from the NIR spectrum is used to produce the test laser beam.

**4.** Method according to one of the preceding claims, **characterized in that** said method is preceded by preliminary tests in which the oscillation time required for a certain cell type and/or a certain substance to be taken up by the cell, is determined as a function of the cell viability, following the application of a series of pulses of predetermined pulse energy.

**5.** Method according to one of the preceding claims, **characterized in that** laser pulses in the ps- or fs-range are applied at a repetition rate of less than 1 MHz.

**6.** Method according to claim 5, **characterized in that** the repetition rate amounts to about 1 kHz.

**7.** Method according to one of the preceding claims, **characterized in that** the pulse energy during the application of the series of pulses is changed from pulse to pulse by controlling an acousto-optic modulator through which the pulse series passes.

**8.** Method according to one of the preceding claims, **characterized in that** pulse energy between 1 and 10,000 nJ is applied.

**9.** Method according to one of the preceding claims, **characterized in that** after the required oscillation time value has been attained a predetermined number of pulses is applied.

**10.** Method according to one of the preceding claims, **characterized in that** the cell is disposed in a medium containing a substance which is to be taken up into the cell and which is chosen from the group consisting of nucleic acids, chromosomes, proteins, organelles, dyes, active pharmaceutical agents and functionalized nanoparticles.

**Revendications**

**1.** Procédé pour l'optoperforation de la membrane cellulaire d'une cellule par application d'impulsions de lumière laser,
**caractérisé par**

- focalisation de la lumière laser pulsée sur la membrane cellulaire à perforer,
- application d'une suite d'impulsions laser d'énergie d'impulsion prédéterminée,
- mesure du temps d'oscillation des bulles se formant au foyer laser à l'aide de la modification d'intensité lumineuse provoquée par les bulles au foyer laser d'un faisceau laser d'essai transmis à travers le foyer laser et
- augmentation de l'énergie d'impulsion jusqu'à une valeur à laquelle le temps d'oscillation des bulles atteint une valeur prédéterminée.

2. Procédé selon la revendication 1, **caractérisé en ce que** seules des modifications d'intensité lumineuse d'une durée de moins de 40 microsecondes sont détectées.

3. Procédé selon une des revendications précédentes, **caractérisé en ce qu'**un laser CW ayant une longueur d'onde d'émission principale issue du spectre NIR est utilisé pour générer le faisceau laser d'essai.

4. Procédé selon une des revendications précédentes, **caractérisé en ce que** le procédé est précédé par des essais préliminaires dans lesquels la valeur de consigne du temps d'oscillation pour un certain type de cellule et/ou une certaine substance à absorber par la cellule est déterminée en fonction de la viabilité de la cellule après application d'une suite d'impulsions ayant une énergie d'impulsion prédéterminée.

5. Procédé selon une des revendications précédentes, **caractérisé en ce que** des impulsions laser dans la gamme ps ou fs sont appliquées avec une fréquence de répétition inférieure à 1 MHz.

6. Procédé selon la revendication 5, **caractérisé en ce que** la fréquence de répétition correspond à peu près à 1 kHz.

7. Procédé selon une des revendications précédentes, **caractérisé en ce que** pendant l'application d'une suite d'impulsions, l'énergie d'impulsion est modifiée d'une impulsion à l'autre par commande d'un modulateur acousto-optique que la suite d'impulsions traverse.

8. Procédé selon une des revendications précédentes, **caractérisé en ce qu'**une énergie d'impulsion comprise entre 1 et 10 000 nJ est appliquée.

9. Procédé selon une des revendications précédentes, **caractérisé en ce qu'**un nombre prédéterminé d'impulsions est appliqué après atteinte de la valeur de consigne du temps d'oscillation.

10. Procédé selon une des revendications précédentes, **caractérisé en ce que** la cellule est disposée dans un milieu qui présente une substance à absorber par la cellule qui est sélectionnée parmi le groupe des acides nucléiques, chromosomes, protéines, organites, colorants, principes actifs pharmaceutiques et nanoparticules fonctionnalisées.

Fig. 1

Fig. 2

FIG. 3

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 10331792 A1

### In der Beschreibung aufgeführte Nicht-Patentliteratur

- **Stevenson et al.** *Optics Express,* 2006, vol. 14 (16), 7125-33

- **Vogel et al.** *Applied Physics B,* 2005, vol. 81, 1015-47